# EUROPÄISCHE PATENTANMELDUNG

(11) **EP 1 757 662 A2**
(43) Veröffentlichungstag der Anmeldung: **28.02.2007**
(21) Anmeldenummer: 06017325.9
(22) Anmeldetag: 22.08.2003
(51) Int. Cl.: C09C 1/00

(54) **Verfahren zur Herstellung von hochorganisierten Kristallen mit Hilfe von Sol-Gel-Methoden**

(30) Priorität: 07.09.2002 DE 10241494; 02.08.2003 DE 20311944 U; 02.08.2003 DE 20311937 U; 02.08.2003 DE 20311939 U; 02.08.2003 DE 20311940 U; 02.08.2003 DE 20311942 U; 02.08.2003 DE 20311943 U
(62) Teilanmeldung aus: 03750426.3
(71) Anmelder: Schott AG, 55122 Mainz (DE)
(72) Erfinder: Beier, Wolfram, 55270 Essensheim (DE); Schnell, Rupert, 67551 Worms (DE)
(74) Vertreter: Sawodny, Michael-Wolfgang

(57) **Zusammenfassung**

Die Erfindung betrifft ein Farbeffekt-Schichtsystem, umfassend ein Trägersubstrat (102), wenigstens eine poröse Materialschicht (101.1, 101.2), die poröse Materialschicht (101.1, 101.2) ist mit dem Trägersubstrat (102) wenigstens mittelbar verbunden, dadurch gekennzeichnet, dass die poröse Materialschicht (101.1, 101.2) eine kristallanaloge oder inverse kristallanaloge Überstruktur aufweist, die von einem regelmäßigen Gefüge aus Partikeln (101) gebildet wird, die charakteristischen Abmessungen der verschiedenen regelmäßig angeordneten Hohlräume der kristallanalogen oder inversen kristallanalogen Überstruktur weitgehend übereinstimmen und innerhalb einer sehr engen Verteilung liegen, wobei diese Verteilung der Verteilung der charakteristischen Abmessungen in photonischen Kristallen entspricht und die kristallanaloge oder inverse kristallanaloge Überstruktur einen regelmäßigen Abstand d aufweist, der derart gewählt ist, dass im wesentlichen Licht einer Wellenlänge im sichtbaren Spektralbereich, d. h. im Bereich 380 nm ≤ d ≤ 780 nm reflektiert wird.

## Beschreibung

Die Erfindung betrifft Materialien mit kristallanaloger Überstruktur, insbesondere photonischen Kristallen, die durch Selbstorganisation und zwar entweder durch Selbstorganisation der Partikel, die den photonischen Kristall ausbilden selbst oder durch Sol-Gel-Infiltration in einen Vorformling, ein sogenanntes Templat erhalten werden; ein Verfahren zur Herstellung derselben sowie die Verwendung derartiger Kristalle.

Photonische Kristalle sind Materialien mit einer kristallanalogen Überstruktur mit einer photonischen Bandlücke, also verbotenen oder unzulänglichen Energiezuständen für Photonen, d. h. Licht bestimmter Frequenz kann sich nicht in alle Raumrichtungen ausbreiten. Photonische Kristalle, die eine derartige optische Bandlücke aufweisen, zeichnen sich durch eine regelmäßige dreidimensionale periodische Gitterstruktur aus, die aus Bereichen mit stark wechselnden Brechungsindizes bestehen. Prinzipiell gibt es verschiedene Verfahren zur Herstellung photonischer Kristalle. Eine Möglichkeit der Herstellung photonischer Kristalle besteht im Einsatz mikromechanischer Verfahren. Hier kann beispielsweise ein Silizium-Wafer mit Siliziumdioxid beschichtet werden, darin gleichmäßige Gräben geritzt und diese mit Polysilizium aufgefüllt werden. Sodann kann die Oberfläche eben geschliffen werden, erneut mit SiO₂ bedeckt und darin ebenfalls regelmäßige Polysiliziumstreifen strukturiert werden, allerdings im rechten Winkel zu dem in der darunterliegenden Schicht. Durch mehrfache Wiederholung können auf diese Art und Weise kreuzweise Doppellagen hergestellt werden. Das SiO₂ kann als Stützmaterial mit Fluorwasserstoff herausgelöst werden, so dass sich eine Kreuzgitterstruktur aus Polysilizium mit regelmäßigen Hohlräumen ergibt. Diesbezüglich wird beispielsweise auf R.Sietmann, "Neue Bauelemente durch photonische Kristalle", Funkschau 26, 1998, S. 76 - 79, oder auf "Silicon-based photonic crystals" by Albert Birner, Ralf B. Wehrspohn, Ulrich M. Gösle und Kurt Busch, Advanced Materials, 2001, 13, Nr. 6, S. 377 - 388 verwiesen.

Eine völlig andere Art der Herstellung photonischer Kristalle ist der Aufbau photonischer Kristalle durch selbstorganisierende oder induziert gesteuerte Prozesse. Derartige selbstorganisierende oder induziert gesteuerte Prozesse sind aus dem Bereich der Kolloidkristalle, die sich beispielsweise aus Titandioxid- oder Siliziumdioxid-Kolloiden oder Polymer-Kolloiden zusammensetzen, bekannt. Die Kolloidkristalle bilden sich spontan unter geeigneten Temperatur- und Druckbedingungen. Sie weisen eine dreidimensionale regelmäßige Überstruktur mit Submikrometer-Periodizität auf. Die Strukturelemente der Kolloidkristalle sind beispielsweise Polymer- z. B. Polystyrolkügelchen mit einer Größe von 10 nm bis 10 µm. Herkömmlicherweise werden derartige kolloidale Kristalle durch Sedimentation hergestellt, die zu dicken polykristallinen Proben innerhalb einer Flüssigkeit führen. Anschließend wird die Flüssigkeit in den sedimentierten kolloidalen Strukturen bestehend aus den zuvor genannten Strukturelementen abgezogen, so dass sich Hohlräume zwischen den Strukturelementen, d.h. beispielsweise den Polymerkügelchen ergeben. Auf diese Art und Weise hergestellte photonische Kristalle weisen Domänen mit einer Größe bis zu einigen Zentimetern (cm) auf.

In einem alternativen Verfahren werden die Kapillarkräfte am Meniskus einer kolloidalen Lösung und eines Substrates dazu verwandt, Kolloide durch Selbstorganisation in dichtgepackte Strukturen zu ziehen. Nachteilig an dem aus dem Stand der Technik bekannten Verfahren zur Herstellung hochorganisierter, insbesondere photonischer, Kristalle mit mikromechanischen Methoden ist der hohe Aufwand.

Bei den bekannten Verfahren, hochorganisierte Kristalle durch Selbstorganisation herzustellen, bestand das Problem darin, dass beim Trocknen der kolloidalen Überstrukturen das Fluid in den Hohlräumen nur sehr schlecht, insbesondere nur über einen sehr langen Zeitraum abgezogen werden konnte.

Photonische Kristalle, die durch Sol-Gel-Infiltration in einen Vorformling, ein sogenanntes Templat, hergestellt werden, neigen zudem dazu, beim Trocknungsprozess in kleine Fragmente zu zerfallen. Dies gilt insbesondere für Proben mit größeren Abmessungen, d.h. einer Schichtdicke größer 1 µm. Derartige Proben können rissig werden oder sogar zu Pulver zerfallen, es sei denn der Trocknungsprozess wird sehr vorsichtig und sehr langsam über Monate hinweg durchgeführt.

Betreffend Sol-Gel-Verfahren, die bei der Sol-Gel-Infiltration eines Vorformlinges eingesetzt werden, zur Erzeugung von Gläsern, Glaskeramiken, Keramiken und Verbundmaterialien wird auf die nachfolgenden Schriften verwiesen:
- Prospects of Sol-Gel-Processes, von Donald R. Ulrich, Journal of Non-Crystalline Solids 100 (1988), pp. 174 - 193,
- Charakterisierung von Si02-Gelen und -Gläsern, die nach der Alkoxid-Gel-Methode hergestellt wurden, von Wolfram Beier, Martin Meier und Günther Heinz Frischat, Glastechische Berichte 58 (1985), Nr. 5, S. 97 - 105
- Glaschemie von Werner Vogel, Springer Verlag, Berlin, Heidelberg, New York, 1992, S. 229 - 233

Bei der Herstellung von hochorganisierten, beispielsweise photonischen, Kristallen durch Sol-Gel-Infiltration, d. h. mittels eines Sol-Gel-Verfahrens wird in einer ersten Stufe des Verfahrens ein Sol gebildet. Sole sind kolloidale Suspensionen von festen und flüssigen Substanzen in einem flüssigen oder gasförmigen Dispersionsmittel, wobei die Partikelgröße zwischen 5 x 10⁻¹⁰ und 2 x 10⁻⁷ m liegt. Da derartige kolloidale Lösungen in der Regel instabil sind, agglomerieren die Teilchen und es entsteht ein Gel. Um einen photonischen Kristall zu erhalten, muss das entstandene Gel getrocknet werden, d. h. die flüssigen Komponenten müssen aus den Hohlräumen des Gels entfernt werden. Beim Trocknen der Gele treten in der Regel enorme Spannungen auf, die das Grundgerüst zerstören können.

Als wellenlängenselektive IR-Blocker oder UV-Blocker werden im Stand der Technik Interferenz-Schichtsysteme verwandt. Dieser Materialien hatten den Nachteil, dass sie sehr aufwendig hergestellt werden mussten. Beispielsweise ist es bei Mehrschichtsystemen für IR-Blocker oder einem 3-Schicht-System für einen UV-Blocker erforderlich, dass ein Substratträger mehrmals hintereinander in unterschiedliche Lösungen eingetaucht wird. Werden die Schichten aufgedampft, beispielsweise mittels eines CVD- oder PVD-Verfahrens, so müssen die Aufdampfmaterialien gewechselt werden.

Als Katalysatorträger werden im Stand der Technik Materialien verwandt, die eine breite Größenverteilung der Hohlräume aufweisen. Derartige Katalysatorträger, beispielsweise aus Zeolithen, weisen einen hohen Strömungswiderstand auf.

Im Stand der Technik werden derzeit zur Immobilisierung von Bakterien und anderen biologischen, mikrobiologischen, bioverfahrenstechnischen sowie medizinischen Aufwendungen und für die Reinigung und Aufbereitung von Wasser beispielsweise offenporige Sintergläser eingesetzt. Derartige Sintergläser werden von der Firma SCHOTT GLAS, Mainz unter dem Markennamen SIRAN® vertrieben und sind beispielsweise im Artikel "Bioreaktoren in der Abwassertechnik" von M. Radke in EntsorgungsPraxis 10/89 beschrieben. Diese Materialien hatten den Nachteil, dass sie stets eine relativ breite Größenverteilung der Hohlräume aufwiesen, so dass die Selektion nicht hochspezifisch gewesen ist.

Lacke als Farbeffekt-Beschichtungen gemäß dem Stand der Technik von Metall-, Glas- oder Plastikoberflächen enthalten meist neben den einfärbenden Pigmenten metallische Partikel, etwa Aluminiumpulver, um einen metallischen Glanz zu erreichen. Durch die Größenvariation dieser Partikel und insbesondere durch eine Zugabe von großen Aluminiumpartikeln und Kunststoffteilchen kann ferner ein Brillanteffekt bewirkt werden. Zur Verstärkung der Farbeffekte und insbesondere zur Erzielung einer irisierenden optischen Wirkung in Verbindung mit einem Perlglanzeffekt können die Farbpigmente blättchenförmig ausgebildet und mit Metall bedampft werden.

Lackbeschichtungen gemäß dem Stand der Technik mit hoher Lichtdynamik, d. h. Lacke mit Glanzeffekten oder solche, die einen Farbeindruck vermitteln, welcher vom Lichteinfall und der Blickrichtung abhängt, zeichnen sich durch eine besonders aufwendige Herstellung und durch eine Begrenzung in der Gestaltung der Farbeffekte aus.

Alternativ können gemäß dem Stand der Technik für transparente oder teiltransparente Materialien, wie etwa Gläser oder Kunststofffolien, Zuschlagsstoffe mit irisierender Farbwirkung bei der Herstellung in das Material eingebaut werden. Ein traditionelles Beispiel hierfür ist das in Regenbogenfarben schillernde, metallisch glänzende Tiffany-Glas aus der Jugendstilzeit, welches meist durch Dämpfe von verschiedentlich gemischten Metallsalzen erzeugt wurde. Nachteilig hierbei ist jedoch, dass sich die Farbwirkung meist nicht präzise steuern lässt, da diese von den Details der Bildung von Metallkolloiden in der Glasmatrix abhängt. Die Einschränkungen, die sich hieraus für die Auswahl der Glasmaterialien und der Herstellungsbedingungen ergeben, sind als nachteilig anzusehen.

Eine Möglichkeit, ohne Pigmentauftrag gemäß dem Stand der Technik eine Oberfläche einzufärben besteht in der Verwendung von Interferenz-Schichtsystemen, die durch eine wellenlängenselektive Reflektion gekennzeichnet sind. Allerdings sind Interferenz-Schichtsysteme aufwendig in der Herstellung, da jede Schicht für sich aufgetragen oder aufgedampft werden muss, ferner ermöglicht die in lediglich eine Richtung alternierende Schichtabfolge eines Interferenz-Schichtsystems nicht die Ausbildung von Farbeffekten. Eine erste Aufgabe der Erfindung ist es, ein Verfahren zur Herstellung von hochorganisierten, insbesondere photonischer Kristallen anzugeben, mit dem die Nachteile des Standes der Technik überwunden werden können, insbesondere soll die Trocknung der selbstorganisierten kristallartigen Überstrukturen und der durch Sol-Gel-Infiltration hergestellten inversen kristallartigen Überstrukturen ohne Schädigung und schneller als im Stand der Technik erfolgen. Insbesonders soll eine Schädigung von nach dem Sol-Gel-Verfahren hergestellten inversen kristallanalogen Überstrukturen beim Trocknen verhindert werden.

Eine weitere Aufgabe der Erfindung ist es, die Nachteile herkömmlicher Materialien, die als IR-Blocker oder UV-Blocker verwandt werden, zu überwinden. Insbesondere sollen IR-Blocker und/oder UV-Blocker beispielsweise auf einfache Art und Weise dadurch hergestellt werden können, dass eine Dispersion, die auf ein Substrat, beispielsweise Fensterglas, einmalig aufgebracht, beispielsweise aufgesprüht oder aufgeschleudert, wird.

Eine weitere Aufgabe der Erfindung besteht darin, die Nachteile herkömmlicher poröser Materialien als Katalysatorträger in chemischen und verfahrenstechnischen Anwendungen, als Material für die Reinigung und Aufbereitung von Wasser sowie zur Immobilisierung von Bakterien und für andere biologische, mikrobiologische, bioverfahrenstechnische sowie medizinische Anwendungen zu überwinden. Insbesondere sollen Katalysatorträger mit niedrigerem Strömungswiderstand zur Verfügung gestellt werden sowie offenporöse Materialien, die so in ihrer Funktionalität beeinflusst werden können, dass gezielt eine Besiedlung mit bestimmten, ausgewählten Mikroorganismen erreicht wird und so diese Mikroorganismen immobilisiert werden können.

Erfindungsgemäß wird die erste Aufgabe dadurch gelöst, dass die hochorganisierten, kristallanalogen Überstrukturen oder inversen kristallanalogen Überstrukturen einer hyperkritischen Trocknung unterzogen werden. Die kristallanalogen Überstrukturen werden auch als Gefüge oder Haufenstruktur bezeichnet.

Durch eine hyperkritische Trocknung wird ein schnelleres Abziehen der Flüssigkeit aus den kristallanalogen Überstrukturen erreicht. Des weiteren wird eine Schädigung der Struktur, insbesondere der inversen Strukturen beim Trocknen verhindert.

Bezüglich der hyperkritischen Trocknung wird auf Fricke J., "Aerogele - eine faszinierende Klasse hochporöser Materialien" in Umschau 1986, Heft 7, S. 374 - 377
sowie
Fricke J., "Aerogels - highly tenuous solids with fascinating properties", Journal of Non-Crystalline Solids 100 (1988), pp. 169 - 173
verwiesen.

Bei der hyperkritischen Trocknung wird der Umstand ausgenutzt, dass oberhalb des kritischen Punktes die Phasengrenze fest/flüssig aufgehoben wird und nun noch eine einzige Phase existiert, d. h. dass oberhalb der kritischen Temperatur beispielsweise ein Gas durch noch so hohen Druck nicht mehr verflüssigt werden kann.

Da das Zerreißen eines Gels beim Trocknen im wesentlichen auf unterschiedlich hohe Kapillarkräfte in den unterschiedlich großen Poren des Gels zurückgeführt werden kann, kann durch eine hyperkritische Trocknung erreicht werden, dass keine Spannungsdifferenzen im zu trocknenden Festkörper mehr auftreten. Hierdurch wird ein Zerreißen des Festkörpers verhindert. Wie zuvor ausgeführt, gibt es oberhalb des kritischen Punktes nur noch eine einzige Phase gasförmig/flüssig und damit keine Kapillarkräfte, die an einer Phasengrenze gasförmig/flüssig auftreten könnten, mehr. Die einzige Phase gasförmig/flüssig kann dann aus den Poren des Gels abgezogen werden, ohne dass der Festkörper beim Trocknen zerstört wird.

Aus der US 5795557 ist die Herstellung von Aerogelen aus Kieselsäure bekannt. In der US 5795557 wird darauf verwiesen, dass Aerogele durch Sol-Gel-Verfahren erhalten werden können. Das Aerogel wird nach Trocknung, d. h. nach Abscheiden des Alkohols erhalten.

In der US 6139626 wird die Herstellung von Templaten, d. h. synthetischen Opalen und die Füllung der Poren des Templats mit kolloidalen Nanokristallen beschrieben. Die kolloidale Nanokristalllösung enthält wenigstens ein Lösungsmittel, welches extrahiert wird.

Die US 6261469 beschreibt dreidimensionale Kristallstrukturen sowohl kleiner als auch größerer Dimensionen, so auch photonischer Kristalle, unter Anwendung von Infiltrationsverfahren und Extraktionsstufen. In der US 6261469 werden beispielsweise aus SiO₂-Kügelchen in kolloidaler Suspension durch Sedimentation synthetische Opale, inverse Opale bzw. Template hergestellt. In diese Template wird dann das gewünschte Material, beispielsweise ein Fluid, infiltriert. Als bevorzugtes Extraktionsverfahren wird auch die superkritische Fluidextraktion in der US 6139626 genannt. Die superkritische Fluidextraktion, wie in der US 6139626 beschrieben, dient lediglich dazu, dass Fluid schneller zu entfernen.

Die hyperkritische Trocknung gemäß einem ersten Aspekt der Erfindung wird aber so geführt, dass eine zerstörungsfreie Trocknung von regelmäßig angeordneten selbstorganisierten oder gesteuert organisierten Partikelanordnungen, insbesondere inverse kristallanaloge Überstrukturen, die durch Sol-Gel-Infiltration hergestellt werden, ermöglicht wird. Als Sol-Gel-Verfahren sich anorganische, organische oder Hybridverfahren wie zum Beispiel das Ormocer-Verfahren denkbar. Die hyperkritische Trocknung kann auch mehrstufig erfolgen, beispielsweise durch Lösemittelaustausch.
Die hyperkritische Trocknung wird insbesondere dafür eingesetzt werden, die selbstorganisierte oder induziert organisierte kristallanaloge Überstrukturen aus beispielsweise Polymerkügelchen zu trocknen, die wiederum als Template für hochbrechende Materialien dienen können. Diese Templaten können mit hochbrechenden Materialien nach der Sol-Gel-Methode infiltriert werden, ergebend eine inverse Kristallstruktur. Durch hyperkritische Trocknung des infiltrierten Gels können schrumpfungsarme, rißfreie inverse photonische Kristalle durch Abformung erhalten werden. Nachdem das hochbrechende Material, das beispielsweise durch Sol-Gel-Infiltration in das Templat eingebracht und gemäß der Erfindung hyperkritisch getrocknet wurde, können die Partikel, die das Templat ausbilden, beispielweise die Polymerkügelchen, zur Vergrößerung des Brechungsindeunterschiedes aus der inversen Kristallstruktur herausgelöst werden Das Herauslösen ist beispielsweise durch Ausbrennen möglich.

Durch das erfindungsgemäße Verfahren wird nicht ein Lösungsmittel schnell extrahiert, wie beispielsweise in der US 6261469 beschrieben, sondern durch hyperkritische Trocknung die Struktur verfestigt und beispielsweise das Templat stabilisiert. Es ist dann beispielsweise möglich, photonische Kristalle oder Template zu erhalten, die ohne Halsbildung stabile, übergeordnete, periodische Strukturen ausbilden. Bislang waren derartige Hälse bei photonischen Kristallen zum Zusammenhalt beispielsweise der Überstrukturen und zur Sicherstellung der mechanischen Stabilität notwendig.

Das Fehlen derartiger Halsansätze aufgrund des erfindungsgemäßen Verfahrens der hyperkritischen Trocknung hat Vorteile betreffend die optischen Eigenschaften und insbesondere, wenn die Partikel, die das Templat ausbilden beispielsweise durch Ausbrennen herausgelöst werden sollen.

Durch das erfindungsgemäße Verfahren wird die Herstellung von optischen Bauteilen mit photonischer Kristallüberstruktur mit großen Abmessungen sowie von dreidimensionalen, optischen Bauteilen mit photonischer Kristallüberstruktur komplexer Form und/oder Strukturierung möglich.

Betreffend die Herstellung von Template, die als Vorformling für die Ausbildung von kristallanalogen Überstrukturen von Festkörpern mit höherem Brechungsindex dienen können und die als sogenannte inverse Opale bezeichnet werden, wird auf "From Opals to Optics: Colloidal Photonic Crystals" von Vicky L. Colvin, MRS Bulletin/August 2001, S. 637-641, verwiesen.

Der Offenbarungsgehalt sämtlicher zuvor genannter Schriften:
- Richard Sietmann "Neue Bauelemente durch photonische Kristalle", Funkschau 26, 1998, S. 76 - 79
- "Silicon-based photonic crystals" by Albert Birner, Ralf B. Wehrspohn, Ulrich M. Gösle und Kurt Busch, Advanced Materials, 2001, 13, Nr. 6, S. 377 - 388
- "From Opals to Optics: Colloidal Photonic Crystals" von Vicky L. Colvin, MRS, Bulletin/August 2001, S. 637-641
- Prospects of Sol-Gel-Processes, von Donald R. Ulrich, Journal of Non-Crystalline Solids 100 (1988), pp. 174 - 193
- Charakterisierung von Si02-Gelen und -Gläsern, die nach der Alkoxid-Gel-Methode hergestellt wurden, von Wolfram Beier, Martin Meier und Günther Heinz Frischat, Glastechische Berichte 58 (1985), Nr. 5, S. 97 - 105
- Glaschemie von Werner Vogel, Springer Verlag, Berlin, Heidelberg, New York, 1992, S. 229 - 233
wird vollumfänglich in den Offenbarungsgehalt der vorliegenden Anmeldung mit aufgenommen.

Eine hyperkritische Trocknung eines Gels kann beispielsweise durch folgende Verfahrensführung im Falle von **T**etra-**m**ethyl-**o**rtho-**s**ilicat Si(OCH₃)₄ (TMOS) zur Herstellung von SiO₂-Aerogelen, die in das Templat zur Ausbildung einer inversen kristallanalogen Überstruktur eingebracht werden, erreicht werden:

Zunächst wird bei konstanter Temperatur der Druck P sehr stark erhöht, beispielsweise bei TMOS zur Herstellung von SiO₂-Aerogelen auf ungefähr 80 bar. Sodann wird bei konstant gehaltenem Druck die Temperatur auf ca. 270° C erhöht. Unter diesen Bedingungen kann das Fluid aus der Gelstruktur herausgedrängt werden, ohne dass die Gelstruktur zusammenbricht oder schrumpft, da eine derartige Prozessführung stets oberhalb der kritischen Temperatur T_{K} erfolgt und nur eine flüssige bzw. gasförmige Phase vorliegt. Das Herausziehen der flüssigen bzw. gasförmigen Phase erfolgt bei Absenken des Druckes auf Atmosphärendruck. Wenn Atmosphärendruck erreicht ist, wird die Temperatur auf Zimmertemperatur erniedrigt.

Mit dem erfindungsgemäßen Verfahren wird eine hyperkritische Trocknung im Gegensatz zur im Stand der Technik beschriebenen superkritischen Fluidextraktion erreicht. Die Verfahrensführung bei der superkritischen Fluidextraktion ist so gewählt, dass das Fluid schnell abgezogen, d. h. extrahiert wird; bei der hyperkritischen Trocknung ist die Verfahrensführung so gewählt, dass die Überstruktur des photonischen Kristalls stabilisiert wird, so dass beispielsweise Halsansätze wie im Stand der Technik bei photonischen Kristallen vermieden werden können und die Überstruktur auch ohne derartige Halsansätze stabil ist.

Überraschenderweise haben die Erfinder herausgefunden, dass Materialien mit einer sehr regelmäßigen Überstruktur, die zu einer optischen Bandlücke führen, nicht nur für optoelektronische Bauteile, einsetzbar sind, sondern sich auch hervorragend in anderen Bereichen der Optik, beispielsweise als Blocker für IR-Strahlung oder UV-Strahlung einsetzen lassen. Ein Grund hierfür liegt in der extrem engen Verteilung der charakteristischen Abmessungen der Hohlräume in photonischen Kristallen, wodurch erreicht werden kann, dass derartige Anordnungen als Linien- bzw. Bandfilter hochwellenlängenselektiv sind.

Hergestellt werden kann ein poröses Material in Form eines photonischen Kristalles, der als IR-Blocker eingesetzt wird, auf verschiedene Art und Weise. In einem ersten Verfahren werden Partikel, beispielsweise Polymer-, Silizumdioxid- oder Titandioxid-Partikel in ein Dispersionsmittel gegeben. Die Partikel organisieren sich im Dispersionsmittel durch langsame Sedimentation zu kristallanalogen Überstrukturen selbst oder induziert gesteuert. Anschließend wird das Dispersionsmittel durch Trocknung, beispielsweise hyperkritische Trocknung, abgezogen und der selbstorganisierte Kristall stabilisiert. Die Selbstorganisation in kristallanalogen Überstrukturen in einem Dispersionsmittel ist insbesondere bei Siliziumdioxid- oder Titandioxid-Partikeln vorteilhaft, da bei derartigen kristallanalogen Überstrukturen ein großer Brechungsindexunterschied zwischen den Partikeln selbst und den luftgefüllten Hohlräumen besteht.

In einem alternativen Verfahren kann der photonische Kristall durch Sol-Gel-Infiltration in einem Vorformling, ein sogenanntes Templat, hergestellt werden.

Photonische Kristalle, die durch Sol-Gel-Infiltration in einen Vorformling, ein sogenanntes Templat, hergestellt werden, neigen zudem dazu, beim Trocknungsprozess in kleine Fragmente zu zerfallen. Dies gilt insbesondere für Proben mit größeren Abmessungen, d. h. einer Schichtdicke größer 1 µm. Derartige Proben können rissig werden oder sogar zu Pulver zerfallen, es sei denn der Trocknungsprozess wird sehr vorsichtig und sehr langsam über Monate hinweg durchgeführt.

In einer besonders bevorzugten Ausführungsform werden die hochorganisierten kristallanalogen Überstrukturen oder inversen kristallanalogen Überstrukturen, die für UV-Blocker oder IR-Blocker eingesetzt werden, einer hyperkritischen Trocknung unterzogen.

Durch eine hyperkritische Trocknung wird eine Schädigung der Struktur, insbesondere der inversen Strukturen beim Trocknen verhindert.

Überraschenderweise haben die Erfinder des weiteren herausgefunden, dass Materialien mit einer sehr regelmäßigen Überstruktur, die zu einer optischen Bandlücke führen, auch in völlig anderen Bereichen als dem Bereich der Optik einsetzbar sind und sich hervorragend als Katalysatorträger in chemischen und verfahrenstechnischen Anwendungen eignen. Insbesondere zeichnen sich Katalysatoren mit derartigen Katalysatorträgern durch einen sehr niedrigen Strömungswiderstand aus. Der Grund hierfür liegt in der extrem engen Verteilung der charakteristischen Abmessungen der Hohlräume in photonischen Kristallen. Des weiteren zeichnen sich derartige Anordnungen aufgrund ihrer engen Verteilung der charakteristischen Hohlräume dadurch aus, dass sie hochselektiv chemische Reaktionen beeinflussen können. Dies ist darauf zurückzuführen, dass die Größe der Hohlräume bzw. die Porengröße den Abmessungen der an der fraglichen Reaktion beteiligten Atome, Moleküle oder Radikale angepasst werden können. Des weiteren ist es durch die Steuerung der Größe der Hohlräume in der krsitallanalogen Überstruktur möglich, die Strömungsgeschwindigkeit exakt einzustellen. Des weiteren kann durch die Verwendung von sogenannten photonischen Kristallen als Katalysatorträger in chemischen und verfahrenstechnischen Anwendungen eine größere katalytische Umsetzungskapazität als die derzeit bekannten ungeordneten porösen Materialien und Haufenwerke erzielt werden. Des weiteren ist es möglich, bei den Kolloidkristallen, die eine optische Bandlücke aufweisen, diese derart gezielt weiterzuentwickeln und zu konditionieren, dass ganz spezielle chemische Reaktionen durch geeignete Gefügeparameter wie Partikelgröße, Partikelform, Partikelabstand, Porösität etc. adressiert werden können.

Hergestellt werden kann ein poröses Material in Form eines photonischen Kristalles, das als Katalysatorträger in chemischen und verfahrenstechnischen Anwendungen eingesetzt wird, beispielsweise mit Hilfe einer langsamten Sedimentation in einem Dispersionsmittel und anschließender hyperkritischer Trocknung.

Durch die hyperkritische Trocknung wird insbesondere eine Schädigung der Struktur verhindert. Bezüglich der hyperkritischen Trocknung wird auf die zuvor gemachten Ausführungen verwiesen.

Die Erfinder haben des weiteren herausgefunden, dass Materialien mit einer sehr regelmäßigen Überstruktur, die zu einer optischen Bandlücke führen, sich hervorragend auch für die Immobilisierung von Bakterien und für andere biologische, mikrobiologische, bioverfahrenstechnische sowie medizinische Anwendungen eignen. Ein Grund hierfür liegt in der extrem engen Verteilung der charakteristischen Abmessungen der Hohlräume in photonischen Kristallen, wodurch erreicht werden kann, dass derartige Anordnungen hochselektiv Bakterien, Viren und andere Mikroorganismen und deren Vorstufen immobilisieren können. Ein weiterer Vorteil der Verwendung von sogenannten photonischen Kristallen für die Immobilisierung von Bakterien und für andere biologische, mikrobiologische, bioverfahrenstechnische sowie medizinische Anwendungen liegt darin, dass derartige regelmäßige Strukturen mit einer größeren Beladungskapazität als die derzeit bekannten untergeordneten porösen Materialien und Haufewerke ausgerüstet werden können. Des weiteren ist es möglich, bei den Kolloidkristallen, die eine optische Bandlücke aufweisen, derart gezielt weiterzuentwickeln und zu konditionieren, dass ganz spezielle Bakterien bzw. Viren durch geeignete Hohlraumgrößen immobilisiert werden können.

Hergestellt werden kann ein poröses Material in Form eines photonischen Kristalles, das zur Immobilisierung von Bakterien und für andere biologische, mikrobiologische, bioverfahrenstechnische sowie medizinische Anwendungen eingesetzt wird, beispielsweise mit Hilfe einer langsamen Sedimentation in einem Dispersionsmittel und anschließender hyperkritischer Trocknung.

Aufgrund der extrem engen Verteilung der charakteristischen Abmessungen der Hohlräume in photonischen Kristallen eignen sich diese Strukturen auch zur Reinigung und Aufbereitung von Wasser. Durch die extrem enge Verteilung der Abmessung der Hohlräume sind derartige Anordnungen hochselektiv. Ein weiterer Vorteil der Verwendung von sogenannten photonischen Kristallen für die Reinigung und Aufbereitung von Wasser liegt darin, dass derartige regelmäßige Strukturen mit einer größeren Durchsatzkapazität als die derzeit bekannten ungeordneten porösen Materialien und Haufenwerke zulassen. Des weiteren ist es möglich, die Kolloidkristalle, die eine optische Bandlücke aufweisen, derart gezielt weiterzuentwickeln und zu konditionieren, dass das Substratmaterial, die Oberflächenbeschaffenheit und die Gefügeparameter, d. h. Partikelgröße, Partikelform, Partikelabstand, Porösität etc., auf die Anwendung hin maßgeschneidert werden können.

Hergestellt werden kann ein poröses Material in Form eines photonischen Kristalles, das für die Reinigung und Aufbereitung von Wasser eingesetzt wird, beispielsweise mit Hilfe einer langsamen Sedimentation in einem Dispersionsmittel und anschließender hyperkritischen Trocknung, bei der das Dispersionsmittel abgezogen wird.

Eine weitere überraschende Verwendung photonischer Kristalle sind FarbeffektSchichten und eine Farbeffekt-Beschichtung. Derartige Schichten bzw. Beschichtungen auf der Basis photonischer Kristalle zeichnen sich durch eine intensive Ausbildung der Farbwirkung sowie der Farbdynamik aus. Die Farbeffekt-Beschichtung ist insbesondere für die Anwendung auf einer Vielzahl von großflächigen und beliebig geformten Substraten geeignet.

Hergestellt werden kann ein poröses, einen Farbeffekt erzeugendes Beschichtungsmaterial in Form eines photonischen Kristalles auf verschiedene Art und Weise. Eine erste erfindungsgemäße Farbeffekt-Beschichtung erhält man dadurch, dass sich Partikel, beispielsweise Polymer-, Siliziumdioxid- oder Titandioxid-Partikel, in ein Dispersionsmittel durch langsame Sedimentation zu kristallanalogen Überstrukturen selbst oder induziert gesteuert organisieren. Hierbei wird die Gitterperiodizität der so entstehenden kristallanalogen Überstruktur durch die Wahl der Partikelgröße bestimmt. Für Farbeffekt-Beschichtungen müssen die kristallanalogen Überstrukturen eine Gitterperiodizität im Brechzahlverlauf im Bereich der Wellenlänge des sichtbaren Spektrums, d.h. im Bereich 380 nm ≤ d ≤ 780 nm, aufweisen. Entscheidend für die optische Qualität der Farbeffekt-Beschichtung ist die strenge Periodizität im Brechzahlverlauf und hohe Symmetrie des photonischen Kristalls.

Die Selbstorganisation in kristallanalogen Überstrukturen in einem Dispersionsmittel ist insbesondere bei Siliziumdioxid- oder Titandioxid-Partikeln vorteilhaft, da bei derartigen kristallanalogen Überstrukturen ein großer Brechungsindexunterschied zwischen den Partikeln selbst und den luftgefüllten Hohlräumen besteht. Zur Schaffung dieser Luft gefüllten Hohlräume muss das Dispersionsmittel aus der kristallanalogen Überstruktur abgezogen werden. Da dies wie voranstehend beschrieben, aufgrund der Flächigkeit der Beschichtung und den wirkenden Kapillarkräften problematisch ist, ist es besonders vorteilhaft, dass das Dispersionsmittels durch eine hyperkritische Trocknung abgezogen wird und so eine hochgeordnete, kristallanaloge Überstruktur mit Luft gefüllten Zwischenräumen entsteht, deren Gitterstruktur so gleichmäßig erhalten bleibt, dass die erwünschten Farbeffekte voll zur Geltung kommen. Die vorteilhafte Wirkdung des Verfahrens mit einer hyperkritischen Trocknung ist darin zu sehen, dass poröse, einen Farbeffekt erzeugende Beschichtungsmaterial in ausreichend stabiler Weise im Wesentlichen ohne die halsartigen, die optischen Eigenschaften der Beschichtung störenden Materialverbindungen zwischen den Partikeln erhalten werden können. Die halsartigen Verbindungen sind beispielsweise für die optischen Eigenschaften des photonischen Kristalls bei Verwendung in Farbeffektschichten störend, da die strenge Periodizität des Filters nachteilig beeinflusst wird.

Eine alternative Farbeffekt-Schicht bzw. eine alternative Farbeffekt-Beschichtung auf der Basis photonischer Kristalle stellt eine inverse Struktur zu der voranstehend beschriebenen dar. Hierzu wird ein photonischer Kristall als Beschichtung durch Sol-Gel-Infiltration in einem Vorformling, ein sogenanntes Templat, hergestellt. Hierbei wird als Template eine erfindungsgemäße, hochgeordnete kristallanaloge Überstruktur ohne halsartige Verbindungen zwischen den die Überstruktur bildenden Partikeln verwendet, wie sie voranstehend beschieben wurde.

Die Erfindung soll nachfolgend noch anhand der Figuren verdeutlicht werden. Es zeigen:
- Fig.1a - c: die Herstellung von kristallanalogen Überstrukturen aus Polymerkügelchen, TiO₂- oder SiO₂ - oder anderen Metalloxid - Kügelchen durch hyperkritische Trocknung
- Fig. 2a - c: die Herstellung von kristallanalogen Überstrukturen aus hochbrechendem Materialien durch Sol-Gel-Infiltration eines Templates und hyperkritische Trocknung des Sol-Gel Infiltrates.
- Fig. 3: eine Farbeffekt-Beschichtung auf einem Substrat umfassend zwei poröse Materialschichten mit unterschiedlicher räumlicher Periodizität,
- Fig. 4: eine kristallanaloge Überstruktur gemäß dem Stand der Technik, wobei zwischen den die Überstruktur bildenden Partikeln halsförmige Materialverbindungen zur mechanischen Verfestigung ausgebildet sind.

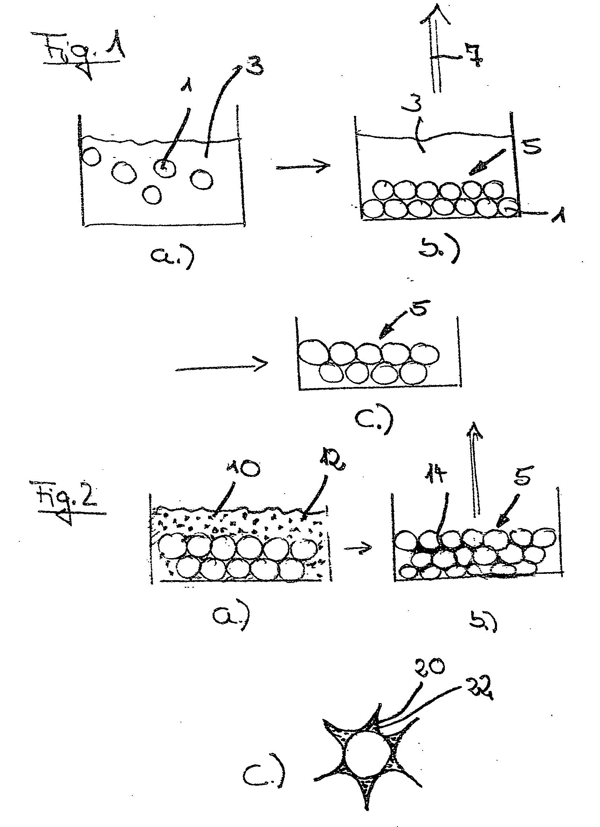

In den Figuren 1a bis 1c ist die Herstellung einer kristallanalogen Überstruktur durch Zugabe von Partikeln 1, vorzugsweise Kügelchen mit Abmessungen 10 nm bis 10 µm in ein Dispersionsmittel 3 und Abziehen des Dispersionsmittels, gezeigt. Die Partikel können Polymer-, TiO₂ - oder SiO₂ - Kügelchen oder Kügelchen aus anderen organischen oder anorganischen Materialien sein. Als Polymerpartikel kommen insbesondere Polystyrol (PS)- oder Polymethylmetacrylat (PMMA)-Partikel, bevorzugt Polystyrol (PS)- oder Polymethylmethacrylat (PMMA)-Kügelchen in Betrecht. Gemäß Figur 1 a sind die Partikel in der Lösung 3 unregelmäßig verteilt. Durch Sedimentaion und Selbstorganisation oder induzierte gesteuerte Organisation ordnen sich die Partikel in kristallanalogen, regelmäßigen Überstrukturen 5 an. Dies ist in Figur 1 b gezeigt. Das in Figur 1 b noch vorhandene Dispersionsmittel wird abgezogen, vorzugsweise durch hyperkritische Trocknung. Es entsteht dann der in Figur 1 c gezeigte Festkörper 5, der eine kristallanaloge Überstruktur aufweist. Der Festkörper 5 kann selbst der photonische Kristall sein, beispielsweise im Falle von TiO₂- oder SiO₂ - Kügelchen oder als Templat für hochbrechende Materialien dienen. Besteht der Festkörper 5 aus TiO₂-Kügelchen, so kann der Festkörper 5 ohne weitere Bearbeitung, beispielsweise Beschichtung, als poröses Material für die Reinigung und Aufbereitung von Wasser verwandt werden.

Ist der Festkörper 5 eine kristallanaloge Überstruktur, beispielsweise aus SiO₂- oder Polymerkügelchen, so bildet der Festkörper 5 das Basismaterial aus, auf das eine TiO₂- oder Titanoxid-haltige Beschichtung aufgebracht werden kann, die dann die funktionelle Schicht für die Reinigung und Aufbereitung von Wasser ausbildet. Insbesondere kann durch die Beschichtung die Funktionalität maßgeschneidert werden.

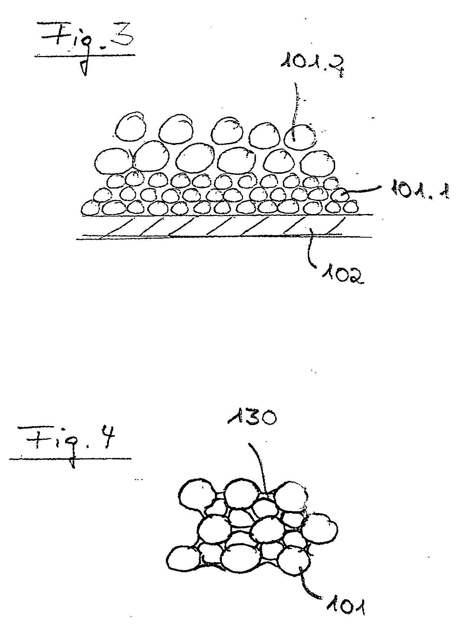

Wird ein Polymerfestkörper mit einer kristallanalogen Überstruktur als Templat verwendet, so kann der photonische Kristall mit hochbrechendem Material wie in Figuren 2a - 2c gezeigt durch Sol-Gel Infiltration mit einem hochbrechenden Material, hergestellt werden. Gemäß Figur 2a wird beispielsweise der Polymerfestkörper mit einer kristallanalogen Überstruktur in eine kolloidale Lösung bzw. Sol 10 gegeben. Die kollidale Lösung umfasst Partikel 12 mit einer Größe zwischen 5·10⁻¹⁰ und 2·10⁻⁷ m, die agglomerieren und eine Gelstruktur ausbilden.

In den Zwischenräumen 14 des Polymerfestkörpers 5, der das Templat für das hochbrechende Material bildet, wird eine Gelstruktur ausgebildet. Die Gelstruktur wird erfindungsgemäß hyperkritisch getrocknet. Die hyperkritisch getrocknete Struktur ist in Figur 2c gezeigt. Das getrocknete hochbrechende Material ist mit 20 bezeichnet, die Mikrostruktur, die sich aufgrund der Mikroporösitäten ergibt mit 22. Um den Brechungsindeunterschied zu erhöhen können die Partikel 1 des Templates herausgelöst werden, beispielsweise bei einem aus Polymerkügelchen aufgebautem Festkörper als Templat durch Herausbrennen.

Im Gegensatz zu einer Anwendung im Bereich der Optik, bei der ein großer Brechnungsindexunterschied zwischen den regelmäßigen Strukturen hergestellt werden muss, ist es bei der Anwendung regelmäßig aufgebauter photonischer Kristalle für die Immobilisierung von Bakterien und anderen biologischen, mikrobiologischen, bioverfarenstechnischen sowie medizinischen Anwendungen nicht notwendig, zwischen den regelmäßigen Strukturen einen hohen Brechungsindexunterschied einzubringen. Dies ist bei photonischen Kristallen sehr aufwendig und wird beispielsweise mit der Methode der inversen Opale erreicht. Nachfolgend soll die Verwendung photonischer Kristalle als FarbeffektSchichten bzw. Beschichtungen beschrieben werden.

Figur 3 zeigt eine Farbeffekt-Beschichtung auf einem Substrat 102 mit zwei porösen, kristallanalog geordneten Schichtlagen 101.1, 101.2 , die sich in ihrer Gitterperiodizität unterscheiden. Beide Gitterperiodizitäten der Brechzahl sollen so gewählt sein, dass nur Licht einer Wellenlänge, die im Bereich des sichtbaren Lichtes, d. h. zwischen 380 nm und 780 nm liegt, reflektiert wird. Da jede der Schichten selektive Wellenlängen reflektiert, entsteht für den Betrachter winkelabhängig ein gemischter Farbeindruck, der gleichzeitig durch eine opaleszierende Wirkung charakterisiert wird. Hierbei kann eine erfindungsgemäße Farbeffekt-Beschichtung jedoch auch durch eine poröse Schicht mit einheitlicher Gitterperiodizität oder mit mehr als zwei unterschiedlichen Gitterperiodizitäten ausgebildet werden.

Die Herstellung einer kristallanalogen Überstruktur ist in den Figuren 1a - 1 c sowie falls beispielsweise ein Polymerfestkörper mit einer kristallanalogen Überstruktur als Templat verwendet wird, in den Figuren 2a - 2c beschrieben.

Figur 4 zeigt in schematisch vereinfachter Weise eine mechanische Verfestigung der kristallanalogen Überstruktur durch die Ausbildung von halsartigen Materialverbindungen 130 zwischen den Partikeln 101. Nachteilig an einer solchen Struktur ist, dass in ihrem Wachstum in der Regel nicht hinreichend genau kontrolliert werden können, so dass sich eine Abweichung von der symmetrischen Struktur und eine Verzerrung des Gitters ergibt, was beispielsweise die Farbeffekte der Beschichtung verringert oder bei anderen Anwendungen andere Nachteile hat, wie beispielsweise eine verringerte Selektorität bei Anwendung im Bereich der Wasseraufbereitung oder bei der Immobilisierung von Mikroorganismen.

Mit der Erfindung wird erstmals ein Verfahren zur Herstellung von hochorganisierten Überstruktur-Materialien angegeben, mit dem photonische Kristalle auch mit relativ großen Abmessungen im Bereich einiger Zentimeter (cm) bis Dezimeter (dm) bei bulk-Materialien und bis zu einigen Metern (m) bei Beschichtungen hergestellt werden können.

Des weiteren gibt die Erfindung poröse Materialien an, die im IR-Wellenbereich hochselektiv als IR-Blocker wirken, beispielsweise als IR-blockendes Beschichtungsmaterial für Fensterscheiben, Automobilscheiben, Brillengläser, technische und wissenschaftliche Bauteile mit IR-Filterfunktion, Bauteile für Solaranlagen, Lampengläser sowie für Elektronikbauteile finden. In Solaranlagen, insbesondere Solarthermieanlagen, gelingt es, mit derartigen IR-Filtern den Wirkungsgrad erheblich zu steigern. Im Bereich der Lampengläser und Abdeckungen kann die Energieausbeute ebenfalls erheblich erhöht werden, da infolge der Reflexion des mit einem IR-blockenden Material beschichteten Substrates das abgestrahlte IR-Licht zurück auf die Lichtquelle, beispielsweise die Wendel, fokussiert wird. Im Bereich der Elektronikbauteile kann ein IR-blockendes Beschichtungsmaterial dafür eingesetzt werden, derartige Bauteile von Wärmestrahlung bzw. zu großer Aufheizung durch benachbarte heiße Komponenten bzw. die Umgebung zu schützen. Die IR-blockende Schicht gemäß der Erfindung kann auf Substrate mit Hilfe eines Tauch-, eines Schleuder- oder Sprühverfahrens aufgebracht werden. Als Substratmaterialien für die IR-blockenden Schichten kommen jede Art von Gläsern, transparenten Grundstoffen oder anderen transparenten Substraten, aber auch opake Substrate wie Metalle und Keramiken in Betracht.

Des weiteren gibt die Erfindung poröse Materialien an, die im UV-Wellenbereich hochselektiv als UV-Blocker wirken, beispielsweise als UV-blockendes Beschichtungsmaterial für Fensterscheiben, Automobilscheiben, Brillengläser, insbesondere Sonnenbrillengläser technische und wissenschaftliche Bauteile mit UV-Filterfunktion finden. Die UV-blockende Schicht gemäß der Erfindung kann auf Substrate mit Hilfe eines Tauch-, eines Schleuder- oder Sprühverfahrens aufgebracht werden. Bevorzugt können wie oben beschrieben die photonischen Kristalle direkt mit Sol-Gel-Methoden hergestellt werden. Als Substratmaterialien für die UV-blockenden Schichten kommen jede Art von Gläsern, transparenten Grundstoffen oder anderen transparenten Substraten, aber auch opake Substrate wie Metalle und Keramiken für reflektive Optiken in Betracht.

Des weiteren wird mit der Erfindung erstmals ein poröses Material und ein Verfahren angegeben, das in bioverfahrenstechnischen und medizinischen Anwendungen, insbesondere Bakterien, hochspezifisch immobilisiert werden können.

Ebenso sind erstmals poröse Materialien und ein Verfahren zur Herstellung angegeben, mit dem Katalysatorträger mit einer sehr regelmäßigen Porengröße hergestellt werden können sowie poröse Materialien mit denen in bioverfahrenstechnischen und medizinischen Anwendungen, insbesondere Bakterien, hochspezifisch immobilisiert werden können.

## Patentansprüche

1. Farbeffekt-Schichtsystem, umfassend
1.1. ein Trägersubstrat (102);
1.2 wenigstens eine poröse Materialschicht (101.1, 101.2);
1.3. die poröse Materialschicht (101.1, 101.2) ist mit dem Trägersubstrat (102) wenigstens mittelbar verbunden;
**dadurch gekennzeichnet, dass**
1.4. die poröse Materialschicht (101.1, 101.2) eine kristallanaloge oder inverse kristallanaloge Überstruktur aufweist, die von einem regelmäßigen Gefüge aus Partikeln (101) gebildet wird;
1.5. die charakteristischen Abmessungen der verschiedenen regelmäßig angeordneten Hohlräume der kristallanalogen oder inversen kristallanalogen Überstruktur weitgehend übereinstimmen und innerhalb einer sehr engen Verteilung liegen,
1.6. wobei diese Verteilung der Verteilung der charakteristischen Abmessungen in photonischen Kristallen entspricht und die kristallanaloge oder inverse kristallanaloge Überstruktur einen regelmäßigen Abstand d aufweist, der derart gewählt ist, dass im wesentlichen Licht einer Wellenlänge im sichtbaren Spektralbereich, d. h. im Bereich 380 nm ≤ d ≤ 780 nm reflektiert wird.

2. Farbeffekt-Schichtsystem nach Anspruch 1, **dadurch gekennzeichnet, dass** die kristallanaloge Überstruktur im Wesentlichen keine halsförmigen Materialverbindungen (130) zwischen den das regelmäßige Gefüge bildenden Partikeln (101) aufweist.

3. Farbeffekt-Schichtsystem nach Anspruch 1, **dadurch gekennzeichnet, dass** die inverse kristallanaloge Überstruktur im Wesentlichen keine inversen halsförmigen Durchgänge in den Wandungen (108) zwischen den Poren (106) aufweist.

4. Farbeffekt-Schichtsystem nach einem der Ansprüche 1 bis 2, **dadurch gekennzeichnet, dass** das poröse Material ein Kolloidkristall mit einer regelmäßigen kristallanalogen Überstruktur ist.

5. Farbeffekt-Schichtsystem nach Anspruch 4, **dadurch gekennzeichnet, dass** der Kolloidkristall ein Polymer- oder ein Titandioxid- oder Siliziumdioxid-Kolloidkristall ist.

6. Farbeffekt-Schichtsystem nach Anspruch 5, **dadurch gekennzeichnet, dass** der Polymer-Kolloidkristall Polystyrol-Partikel oder Polymethylmetacrylat-Partikel umfasst.

7. Farbeffekt-Schichtsystem nach einem der Ansprüche 1 bis 6, **dadurch gekennzeichnet, dass** das poröse Material ein hochbrechendes Material ist, das durch Abformen der Struktur eines Kolloidkristall als inverse kristallanaloge Überstruktur erhalten wird.

8. Farbeffekt-Schichtsystem nach Anspruch 7, **dadurch gekennzeichnet, dass** das hochbrechende Material eines der nachfolgenden Materialien ist:
TiO₂
InP
CdSe

9. Farbeffekt-Schichtsystem nach einem der Ansprüche 7 bis 8, **dadurch gekennzeichnet, dass** die inverse kristallanaloge Überstruktur durch Sol-Gel-Infiltration des Kolloidkristalls mit hochbrechendem Material hergestellt wird.

10. Farbeffekt-Schichtsystem nach Anspruch 9, **dadurch gekennzeichnet, dass** die mittels Sol-Gel-Infiltration hergestellte inverse kristallanaloge Überstruktur hyperkritisch getrocknet wird.
